# EUROPÄISCHE PATENTSCHRIFT

(11) **EP 0 569 792 B1**
(45) Veröffentlichungstag und Bekanntmachung des Hinweises auf die Patenterteilung: **21.02.1996**
(21) Anmeldenummer: 93106985.0
(22) Anmeldetag: 29.04.1993
(51) Int. Cl.: C07C 231/02, C07C 213/02, C07C 233/60, C07C 215/76

(54) **Verfahren zur Herstellung von N-acylierten p-Aminophenolen**
Process for the preparation of N-acylated p-aminophenols
Procédé de préparation d'aminophénols N-acylés

(30) Priorität: 12.05.1992 DE 4215592
(43) Veröffentlichungstag der Anmeldung: 18.11.1993
(73) Patentinhaber: BAYER AG, D-51368 Leverkusen (DE)
(72) Erfinder: Landscheidt, Heinz, Dr., W-4100 Duisburg 1 (DE); Klausener, Alexander, Dr., W-5000 Köln (DE); Blank, Heinz-Ulrich, Dr., W-5068 Odenthal-Glöbusch (DE)

(56) Entgegenhaltungen:
- US-A- 2 998 450
- US-A- 3 917 695
- METHODEN DER OGANISCHEN CHEMIE (HOUBEN-WEYL) Bd. VI/1C , 1976 , STUTTGART Seiten 91 - 101 'Phenole (Teil 1)'

## Beschreibung

Die Erfindung betrifft ein Verfahren zur Herstellung N-acylierter p-Aminophenole durch katalytische Hydrierung aromatischer Nitroverbindungen mit freier p-Stellung im Sinne einer Bamberger-Reaktion und anschließender Acylierung der entstandenen Aminogruppe.

N-acylierte p-Amino-phenole sind als Schädlingsbekämpfungsmittel, insbesondere als Fungizide, bekannt (EP 339 418).

Die Herstellung N-acylierter p-Amino-phenole erfolgt üblicherweise durch Acylierung der entsprechenden freien p-Amino-phenole in Gegenwart von Verdünnungsmitteln und unter Zuhilfenahme basischer Hilfsmittel (EP 339 418). Die benötigten p-Amino-phenole sind prinzipiell bekannte Verbindungen und werden im allgemeinen durch Nitrieren von in p-Stellung unsubstituierten Hydroxyaromaten sowie anschließende Reduktion der Nitrogruppe oder durch Reduktion in p-Stellung unsubstituierter Nitroaromaten im Sinne einer Bamberger-Reaktion hergestellt (Houben-Weyl, 4.Aufl., Bd. VI/1c; (1976), S. 85-117). In beiden Fallen ist es üblich und erforderlich, die als Zwischenprodukte für die Herstellung der gewünschten N-acylierten p-Amino-phenole benötigten freien p-Amino-phenole zu isolieren und in gereinigter Form weiter umzusetzen. Diese Vorgehensweise ist umständlich, unwirtschaftlich, mit Ausbeuteverlusten und hohem Anfall an Abfällen, insbesondere Abwässern verbunden. Im übrigen ist die Nitrierung von Phenolen unter reaktions- und sicherheitstechnischen Gesichtspunkten bedenklich.

Aus US 2.998.450 ist ein Verfahren zur Herstellung von N-Acetyl-p-aminophenol bekannt, bei dem in "direkter" Weise zunächst Nitrobenzol in konz. H₂SO₄ elektrochemisch reduziert wird und das entstandene p-Aminophenol ohne Zwischenisolierung mit Na-acetat/Acetanhydrid weiter umgesetzt wird. Das elektrochemisch hergestellte Reduktionsgemisch muß jedoch mit großen Mengen CaCO₃ von der Schwefelsäure befreit werden und mit Hilfe von Benzol-Extraktionen, Behandlung mit A-Kohle und weiterer Behandlung mit NaHSO₃ oder Na₂SO₃ auf die Acetylierung vorbereitet werden. Die A-Kohle wird durch Regenerierung mit heißer Lauge und heißer Säure wiederverwendungsfähig gemacht.

Es bestand daher die Aufgabe, ein Verfahren zur Herstellung der obengenannten N-acylierten p-Amino-phenole zu finden, das mehrstufige und aufwendige Reaktionssequenzen vermeidet, die Nitrierung von Hydroxyaromaten vermeidet und das in guten Ausbeuten zu Produkten hoher Reinheit führt.

Diese Aufgabe wird durch das erfindungsgemäße Verfahren ohne Zwischenisolierung des p-Aminophenols gelöst.

Die vorliegende Erfindung betrifft ein Verfahren zur Herstellung N-acylierter p-Amino-phenole der allgemeinen Formel 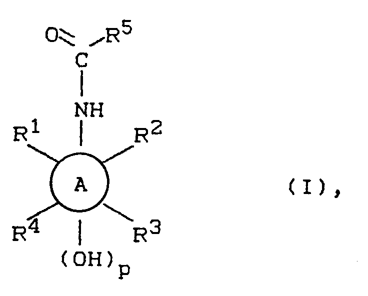
in der
- A: für den Benzol- oder den Naphthalinkern steht,
- p: die p-Stellung zur Aminogruppe anzeigt,
- R¹, R², R³ und R⁴: unabhängig voneinander Wasserstoff, C₁-C₄-Alkyl, C₁-C₄-Alkoxy, Halogen oder die Gruppe CO-R⁶ bedeuten, in welcher R⁶ für Wasserstoff, C₁-C₄-Alkyl, C₁-C₄-Alkoxy, Hydroxy oder gegebenenfalls substituiertes Aryl steht, und
- R⁵: C₁-C₂₀-Alkyl, C₃-C₁₀-Cycloalkyl, C₅-C₁₀-Cycloalkenyl, C₁-C₁₀-Alkoxy, gegebenenfalls substituiertes Aryloxy oder gegebenenfalls substituiertes Aryl bedeutet,
das dadurch gekennzeichnet ist, daß Nitroverbindungen der allgemeinen Formel 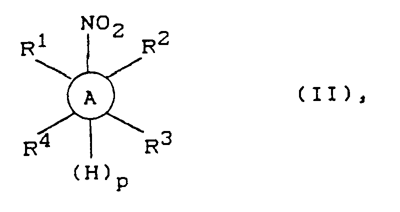
in der
R¹ bis R⁴, A und p die obige Bedeutung besitzen, in einem wäßrig-sauren Reaktionsmedium, in welchem die Wassermenge das 2- bis 40-fache, bevorzugt das 3-bis 30-fache, besonders bevorzugt das 4- bis 20-fache der Gewichtsmenge der aromatischen Nitroverbindung beträgt, und gegebenenfalls in Gegenwart eines mit Wasser mischbaren organischen Lösungsmittels unter Zuhilfenahme eines Katalysators aus der Gruppe der Platinmetalle im Sinne einer Bamberger-Reaktion bei einer Temperatur von 50-160°C, bevorzugt 70-140°C, besonders bevorzugt 90-120°C, und einem Wasserstoff-Partialdruck von 0,1-50 bar, bevorzugt 0,5-30 bar, besonders bevorzugt 1-20 bar, hydriert werden und das erhaltene Reaktionsgemisch mit einer Verbindung der Formel 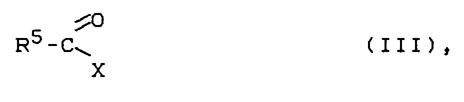
wobei
- R⁵: die obige Bedeutung hat und
- X: für eine Fluchtgruppe steht,
in einer Menge von 0,5-2 Mol, bevorzugt 0,8-1,4 Mol pro Mol der eingesetzten Nitroverbindung, bei einer Temperatur von -30°C bis +150°C, bevorzugt von -20°C bis +120°C, besonders bevorzugt von -10°C bis +100°C, und gegebenenfalls in Gegenwart eines basischen Hilfsmittels oder eines anderen Säureakzeptors und gegebenenfalls in Gegenwart eines Verdünnungsmittels umsetzt.

Überraschender Vorteil des erfindungsgemäßen Verfahrens ist es, daß das bei der katalytischen Hydrierung p-unsubstituierter Nitroaromaten unter wäßrig-sauren Bedingungen im Sinne einer Bamberger-Reaktion erhaltene Reaktionsgemisch glatt mit Acylierungsmitteln der allgemeinen Formel (III) umgesetzt werden kann, wobei die gewünschten Produkte der allgemeinen Formel (I) erhalten werden, ohne daß Ausbeuteverluste, bedingt durch Hydrolyse der genannten Acylierungsmittel der Formel (III) zu beobachten wären.

Ein weiterer überraschender Vorteil des erfindungsgemäßen Verfahrens ist es, daß die gewünschten Produkte der allgemeinen Formel (I) in hohen Reinheiten erhalten werden, obwohl auf eine Isolierung und Zwischenreinigung der als Zwischenprodukte auftretenden p-Amino-phenole, wie sie Stand der Technik ist, verzichtet wird.

Weitere Vorteile sind:
- die Verringerung der Abwasserbelastung gegenüber dem bisherigen Prozeß
- die Erhöhung der Wirtschaftlichkeit durch Verringerung der Anzahl der erforderlichen Verfahrensschritte.

C₁-C₄-Alkyl ist beispielsweise Methyl, Ethyl, Propyl, 2-Propyl, Butyl, 2-Butyl, 2-Methyl-propyl (Isobutyl), 2-Methyl-2-propyl (tert.-Butyl); weiteres Alkyl mit bis 20 C-Atomen ist beispielsweise Pentyl, 2-Pentyl, 3-Pentyl, 2,2-Dimethyl-propyl (Neopentyl), 2-Methyl-2-butyl, 3-Methyl-2-butyl, Hexyl, 2-Hexyl, 3-Methyl-3-pentyl, 3,3-Dimethyl-2-butyl, 2,2-Dimethyl-butyl, Heptyl, Octyl, Nonyl, Decyl, Pentadecyl, Hexadecyl, Heptadecyl, Octadecyl, Nonadecyl und Eicosyl. Die Substituenten R¹, R², R³, R⁴ und R⁶ bedeuten unabhängig voneinander bevorzugt Methyl und Ethyl, besonders bevorzugt Methyl. Der Substituent R⁵ bedeutet bevorzugt geradkettiges oder verzweigtes Alkyl mit bis zu 8 Kohlenstoffatomen der oben genannten Art, besonders bevorzugt verzweigtes Alkyl mit bis zu 6 Kohlenstoffatomen der oben genannten Art.

C₁-C₄-Alkoxy ist über ein Sauerstoffatom gebundenes geradkettiges oder verzweigtes Alkyl der oben genannten Art; entsprechend ist Alkoxy mit bis zu 10 C-Atomen zu verstehen. Die Substituenten R¹, R², R³, R⁴ und R⁶ bedeuten unabhängig voneinander bevorzugt C₁-C₂-Alkoxy, besonders bevorzugt Methoxy. Der Substituent R⁵ bedeutet bevorzugt geradkettiges oder verzweigtes Alkoxy mit bis zu 8 Kohlenstoffatomen, besonders hevorzugt geradkettiges oder verzweigtes Alkoxy mit bis zu 6 Kohlenstoffatomen.

C₃-C₁₀-Cycloalkyl bedeutet gesättigte, gegebenenfalls substituierte, vorzugsweise 3- bis 8-gliedrige, besonders bevorzugt 3- bis 6-gliedrige carbocyclische Ringsysteme, wobei als Substituenten beispielhaft Halogen, vorzugsweise Fluor oder Chlor sowie Niederalkyl mit 1 bis 4 Kohlenstoffatomen, vorzugsweise Methyl, genannt seien. Insbesondere genannt seien dabei Cyclopropyl, 1-Methyl-cyclopropyl, 1-Fluor-cyclopropyl, 1-Chlor-cyclopropyl, Cyclobutyl, 1-Methyl-cyclobutyl, Heptafluor-cyclobutyl, Cyclopentyl, 1-Methyl-cyclopentyl, 2,2-Dimethyl-cyclopentyl, Cyclohexyl, 1-Methyl-cyclohexyl, 4,4-Dimethyl-cyclohexyl, 4-tert.-Butyl-cyclohexyl und 1-Adamantyl.

C₅₋₁₀-Cycloalkenyl bedeutet einfach oder mehrfach ungesättigte, nicht-aromatische, gegebenenfalls substituierte, vorzugsweise 5- bis 8-gliedrige, besonders bevorzugt 5- und 6-gliedrige carbocyclische Ringsysteme, wobei als Substituenten beispielhaft Halogen, vorzugsweise Fluor oder Chlor sowie Niederalkyl mit 1 bis 4 Kohlenstoffatomen, vorzugsweise Methyl, genannt seien. Insbesondere genannt seien 1-, 2- und 3-Cyclopentenyl, 1-, 2-, 3- und 4-Cyclohexenyl, 2-Methyl-2-cyclopentenyl, 3-Methyl-3-cyclopentenyl, 2-Methyl-2-cyclohexenyl, 2-Chlor-2-cyclohexenyl, 1-Methyl-4-cyclohexenyl und 1,5-Dimethyl-5-cyclohexenyl.

Aryl bedeutet gegebenenfalls substituiertes Phenyl mit bis zu 5, bevorzugt bis zu 4, besonders bevorzugt bis zu 3 Substituenten, die beliebige Positionen in ortho-, meta-oder para-Stellung zueinander einnehmen können. Als Substituenten bevorzugt sind Halogen, insbesondere Fluor oder Chlor, C₁-C₄-Alkyl, bevorzugt Methyl, Halogenalkyl, bevorzugt Trifluormethyl und Difluormethyl, C₁-C₄-Alkoxy, bevorzugt Methoxy und Ethoxy, Halogenalkyloxy, bevorzugt Trifluormethoxy und Tetrafluorethoxy sowie C₁-C₄-Alkoxycarbonyl, bevorzugt Methoxycarbonyl und Ethoxycarbonyl.

Aryloxy bedeutet über ein Sauerstoffatom verknüpftes gegebenenfalls substituiertes Phenyl mit bis zu 5, bevorzugt bis zu 4, besonders bevorzugt bis zu 3 Substituenten, die beliebige Positionen in ortho-, meta- oder para-Stellung zueinander einnehmen können. Als Substituenten bevorzugt sind Halogen, insbesondere Fluor oder Chlor, C₁-C₄-Alkyl, bevorzugt Methyl, Halogenalkyl, bevorzugt Trifluormethyl und Difluormethyl, C₁-C₄-Alkoxy, bevorzugt Methoxy und Ethoxy, Halogenalkyloxy, bevorzugt Trifluormethoxy und Tetrafluorethoxy sowie Alkoxycarbonyl, bevorzugt Methoxycarbonyl und Ethoxycarbonyl.
Halogen bedeutet beispielsweise, Fluor, Chlor oder Brom, bevorzugt Fluor oder Chlor.

Die Fluchtgruppe X steht beispielsweise für Halogen, bevorzugt für Fluor, Chlor oder Brom, besonders bevorzugt für Chlor, für den Rest O-R⁵ oder für den Rest O-CO-R⁵, in dem R⁵ die oben angegebene Bedeutung hat. Darüber hinaus sind grundsätzlich alle Fluchtgruppen X, die eine hinreichende Aktivierung des Restes CO-R⁵ im Sinne einer Acylierung gewährleisten, geeignet, beispielsweise Alkoxy.
Die Durchführung des erfindungsgemäßen Verfahrens sei an folgendem Beispiel erläutert: 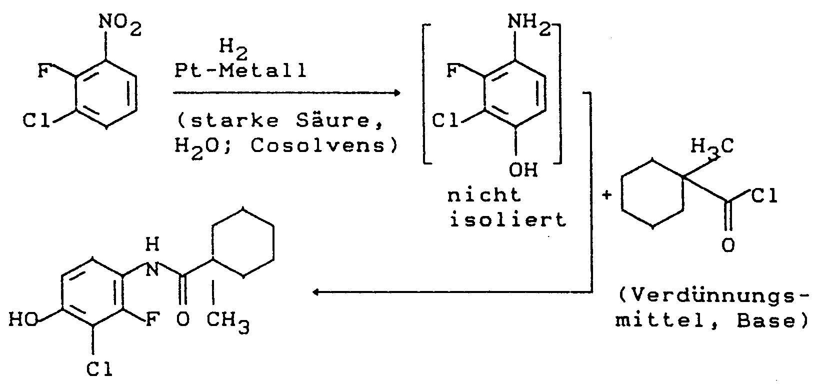
Als gegebenenfalls verwendete Cosolventien im Sinne der vorliegenden Erfindung sind prinzipiell alle unter den zur Anwendung kommenden Reaktionsbedingungen stabilen organischen, mit Wasser mischbaren Lösungsmittel, die die katalytische Hydrierung nicht negativ beeinflussen, geeignet. Solche Cosolventien sind beispielsweise niedere Alkohole und Polyole mit 1 bis 4 C-Atomen, wie Methanol, Ethanol, n- und i-Propanol, n-, i-, sec- und tert.-Butanol, Ethylenglykol, 1,2- und 1,3-Propandiol, 1,2-, 1,3-, 1,4- und 2,4-Butandiol sowie 1,2,3-Propantriol. Weitere Beispiele sind wasserlösliche Ether der genannten Polyole wie Glykolmono- und -dimethylether, Glykolmono- und -diethylether. Beispiele für weitere erfindungsgemäß einsetzbare Cosolventien sind wasserlösliche cyclische Ether, wie Tetrahydrofuran und Dioxan, mit Wasser mischbare Ketone, wie Aceton und Methylethylketon, wasserlösliche Carbonamide, insbesondere die am Stickstoffatom 2-fach alkylierten, wie N,N-Dimethylacetamid, N,N-Dimethylformamid und die entsprechenden ethylierten niederen Carbonsäureamide, sowie niedere aliphatische Carbonsäuren mit 1 bis 4 C-Atomen, wie Ameisensäure, Essigsäure oder Propionsäure. In bevorzugter Weise seien die genannten niederen Alkohole, Ethylenglykol und seine Mono- und Dimethylether der genannten Art und Dioxan genannt. Besonders bevorzugt sei Methanol, Ethanol, Ethylenglykol, Glykolmono- und -dimethylether sowie Dioxan genannt. Die genannten Cosolventien können sowohl einzeln als auch als Gemisch mehrerer von ihnen eingesetzt werden.

Die Menge des gegebenenfalls verwendeten organischen, mit Wasser mischbaren Lösungsmittels beträgt das 0,01-bis 3-fache, bevorzugt das 0,03- bis 2-fache, besonders bevorzugt das 0,05- bis 1-fache der Gewichtsmenge der aromatischen Nitroverbindung.

Für das wäßrig-saure Reaktionsmedium kommen starke anorganische oder organische Säuren in Betracht, beispielsweise Schwefelsäure, Phosphorsäure, Salpetersäure, Salzsäure, Perchlorsäure, Methansulfonsäure, Toluolsulfonsäure, Perfluormethansulfonsäure und andere dem Fachmann bekannte. In bevorzugter Weise werden Schwefelsäure oder eine der genannten Sulfonsäuren eingesetzt; in besonders bevorzugter Weise wird Schwefelsäure eingesetzt.

Die Menge der starken Säuren beträgt 0,4 bis 10 Äquivalente, bevorzugt 0,5 bis 2 Äquivalente, besonders bevorzugt 0,5 bis 1,2 Äquivalente, bezogen auf 1 Mol der aromatischen Nitroverbindung.

Die Wassermenge für das wäßrig-saure Reaktionsmedium beträgt das 2- bis 40-fache, bevorzugt das 3- bis 30-fache, besonders bevorzugt das 4- bis 20-fache der Gewichtsmenge der aromatischen Nitroverbindung.

Das erfindungsgemäße Verfahren wird bei einer Temperatur von 50 bis 160°C, bevorzugt 60 bis 140°C, besonders bevorzugt 70 bis 120°C durchgeführt.

Zur katalytischen Hydrierung im Rahmen des erfindungsgemäßen Verfahrens wird unter erhöhtem Druck gearbeitet, wozu in einem Druckreaktor beliebiger Art, wie er dem Fachmann bekannt ist, gearbeitet wird. Selbstverständlich ist ein solcher Reaktor säurefest unter den erfindungsgemäß einzustellenden Reaktionsbedingungen. Als erhöhter Druck sei ein solcher von 2 bar bis zu 50 bar genannt. An diesem erhöhten Druck ist der Wasserstoff-Partialdampfdruck mit 0,1 bis 50 bar, bevorzugt 0,5 bis 30 bar, besonders bevorzugt 1 bis 20 bar beteiligt, kann somit also auch den Gesamtdruck von bis zu 50 bar ausmachen. Die Differenz zwischen dem Wasserstoff-Partialdampfdruck und dem Gesamtdruck ist im allgemeinen der Eigendruck des Reaktionssystems, also der Dampfdruck des Wassers und des zuzusetzenden organischen, mit Wasser mischbaren Lösungsmittels. Hinzu tritt der Dampfdruck der Nitroverbindung. Für den reibungslosen Ablauf einer katalytischen Hydrierung ist weiterhin nach dem Befüllen des Druckreaktors mit den umzusetzenden Stoffen und dem Reaktionsmedium ein Spülen mit Inertgas, wie Stickstoff, Edelgas usw. erforderlich. Auch ein nach Verschließen des Druckreaktors darin verbliebener Rest des inerten Spülgases trägt zum gesamten Druck bei. Im allgemeinen wird hierzu so verfahren, daß der verschlossene Druckreaktor auf die gewünschte Reaktionstemperatur gebracht wird, bevor der Wasserstoff-Partialdampfdruck durch Aufdrücken von Wasserstoff eingestellt wird. Wasserstoff wird dann solange nachgesetzt, wie er durch das Reaktionsgemisch aufgenommen wird.

Als Katalysator für das erfindungsgemäße Verfahren kommen Edelmetalle der Platingruppe, insbesondere Platin und/oder Palladium, in Frage. In gleicher Weise können Verbindungen der Platinmetalle, beispielsweise Platin- und/oder Palladiumverbindungen, eingesetzt werden. Diese Verbindungen werden sodann vom Hydrierwasserstoff zum hydrieraktiven Platinmetall reduziert. Das Platinmetall oder eine Verbindung des Platinmetalls kann mit oder ohne Träger verwendet werden. Träger können beispielsweise Silikagel, Aluminiumoxid, Zeolithe, Molsiebe, Kohle oder andere dem Fachmann bekannte Träger, bevorzugt Kohle, sein. Für den Fall der Mitverwendung eines Trägers beträgt die Metallbelegung 0,05 bis 8 Gew.-%, bevorzugt 0,1 bis 6 Gew.-%, besonders bevorzugt 0,25 bis 5 Gew.-% des Gesamtkatalysators. Der Katalysator mit oder ohne Träger wird in einer solchen Menge eingesetzt, daß 0,001 bis 0,3 Gew.-%, bevorzugt 0,005 bis 0,1 Gew.-%, besonders bevorzugt 0,01 bis 0,1 Gew.-% des Platinmetalls, bezogen auf die umzusetzende Nitroverbindung, vorliegen.

Das gesamte Reaktionsgemisch wird während der Wasserstoffaufnahme intensiv durchmischt. Hierzu ist der Druckreaktor mit einem Rührer oder mit einer Hubeinrichtung ausgestattet oder als Schüttelautoklav ausgeführt.

Kennzeichnend für das erfindungsgemäße Verfahren ist es, daß die verwendeten Katalysatoren nach Beendigung des Reduktionsschrittes oder auch nach Beendigung der gesamten Reaktionssequenz, bevorzugt nach Beendigung des Reduktionsschrittes, in unverändert aktiver oder nahezu unverändert aktiver Form wiedergewonnen werden können.

Die Isolierung des Katalysators nach Beendigung des Reduktionsschrittes bzw. der gesamten Reaktionssequenz geschieht durch Filtration, Abdekantieren oder Zentrifugieren vom restlichen Reaktionsgemisch.

Erfolgt die Abtrennung und Wiedergewinnung des Katalysators unmittelbar nach dem Reduktionsschritt, so kann es gegebenenfalls von Vorteil sein, ein gegebenenfalls im Zuge der Durchführung dieses Reduktionsschrittes eingesetztes Cosolvens ganz oder teilweise aus dem Reaktionsgemisch zu entfernen. Dies kann unter erhöhtem oder vermindertem Druck oder unter Normaldruck durch Destillation geschehen, bevorzugt durch Destillation unter vermindertem Druck.

Als Verdünnungsmittel zur Durchführung des 2. Reaktionsschrittes des erfindungsgemäßen Verfahrens kommen unter den Reaktionsbedingungen inerte organische Lösungsmittel in Frage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Der 2. Reaktionsschritt des erfindungsgemäßen Verfahrens kann somit auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-C₁₃/C₁₅-alkylammoniumchlorid, Dibenzyldimethyl-ammoniummethylsulfat, Dimethyl-C₁₂/C₁₄-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Als basisches Hilfsmittel für die Durchführung des 2. Reaktionsschrittes des erfindungsgemäßen Verfahrens kommen alle üblicherweise verwendbaren anorganischen und organischen Basen in Frage. Vorzugsweise verwendet man Alkalimetall- oder Erdalkalimetallhydroxide, -carbonate oder -hydrogencarbonate, wie beispielsweise Kaliumhydroxid, Calciumhydroxid, Natriumhydroxid, Kaliumcarbonat, Calciumcarbonat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des 2. Reaktionsschrittes des erfindungsgemäßen Verfahrens in einem größeren Bereich variiert werden. Im allgemeinen arbeitet man bei Temperaturen zwischen -30°C und +150°C, vorzugsweise bei Temperaturen zwischen -20°C und +120°C, besonders bevorzugt bei Temperaturen von -10°C bis +100°C.

Der 2. Teilschritt des erfindungsgemäßen Verfahrens wird im allgemeinen unter Normaldruck durchgeführt. Es ist jedoch auch möglich, ihn unter vermindertem oder erhöhtem Druck durchzuführen.

Zur Durchführung des 2. Teilschrittes des erfindungsgemäßen neuen Verfahrens setzt man pro Mol des als Ausgangsstoff verwendeten Nitroaromaten im allgemeinen 0,5 bis 2,0 Mol des Acylierungsreagenzes (z.B. des Säurechlorides) ein, bevorzugt 0,8 bis 1,4 Mol sowie 2 bis 5 Mol des basischen Hilfsmittels (z.B. Natriumhydroxid), bevorzugt 3 bis 4 Mol.

Die Reaktionsdurchführung, Aufarbeitung und Isolierung der Reaktionsprodukte erfolgt nach allgemein üblichen Methoden (vgl. auch die Herstellungsbeispiele).

Das erfindungsgemäße Verfahren wird mit einem Nitrobenzol oder einem 1-Nitro-naphthalin durchgeführt, das gemäß Formel (II) substituiert sein kann. In bevorzugter Weise wird es mit einem Nitrobenzol durchgeführt, das gemäß Formel (II) substituiert sein kann.

Wichtige aromatische Nitroverbindungen als Ausgangsmaterialien für das erfindungsgemäße Verfahren sind: Nitrobenzol, 2-Nitrotoluol, 3-Nitrotoluol, 2-Chlornitrobenzol, 3-Chlor-nitrobenzol, 2-Ethyl-nitrobenzol, 3-Ethyl-nitrobenzol, 2-Acetyl-nitrobenzol, 3-Acetyl-nitrobenzol, 2-Nitrobenzoesäure und Ester, 3-Nitrobenzoesäure und Ester, 2-Fluor-nitrobenzol, 3-Fluor-nitrobenzol, 2,3-Dichlor-nitrobenzol, 2,3-Difluor-nitrobenzol, 2-Chlor-3-fluor-nitrobenzol, 2-Fluor-3-chlor-nitrobenzol, 1-Nitronaphthalin, 2-Chlor-3-methyl-nitrobenzol, 2-Methyl-3-chlor-nitrobenzol.

Aus solchen Nitroverbindungen entstehen erfindungsgemäß die korrespondierenden p-Aminophenole, bevorzugt die der Benzolreihe, die sodann erfindungsgemäß acyliert werden.

In bevorzugter Weise seien folgende aromatische Nitroverbindungen der Benzolreihe genannt, aus denen die korrespondierenden und anschließend zu acylierenden p-Aminophenole der Benzolreihe entstehen: 2,3-Dichlornitrobenzol, 3-Nitrobenzoesäure, 2-Fluor-nitrobenzol, 3-Fluornitrobenzol, 2-Nitrotoluol, 3-Nitrotoluol, 2-Chlor-nitrobenzol, 3-Chlor-nitrobenzol, 2-Nitrobenzoesäure, 2-Chlor-3-fluor-nitrobenzol, 2-Fluor-3-chlor-nitrobenzol, 2-Chlor-3-methyl-nitrobenzol und 2-Methyl-3-chlor-nitrobenzol.

### Beispiel 1

In einem 1,3 l Emailleautoklaven wurden 96 g (0,5 Mol) 2,3-Dichlornitrobenzol, 20 ml Dimethoxyethan (DME), 400 ml Wasser, 60 g 98 %ige (0,6 Mol) Schwefelsäure sowie 1 g Platin-Katalysator (5 % auf Aktivkohle) vorgelegt und unter starkem Rühren auf 105°C erhitzt.

Danach wurde 10 bar Wasserstoff aufgedrückt und bis zur Druckkonstanz hydriert.

Die Reaktionsmischung wurde mit 200 ml Wasser versetzt und heiß filtriert.

Am Rotationsverdampfer wurden 150 ml DME/Wassergemisch abdestilliert und die erhaltene Suspension in einem Vierhalskolben mit Rührer, Rückflußkühler, Tropftrichter und pH-Meßelektrode übergeführt.

Es wurde mit 20 %iger Natronlauge auf pH 5,5 gestellt, mit 250 ml Toluol versetzt und auf 50°C erwärmt.

30,5 g (0,19 Mol) 1-Methylcyclohexancarbonsäurechlorid wurden innerhalb 30 min zugetropft, 10 min nachgerührt und mit 20 %iger Natronlauge pH 4,5 eingestellt.

Anschließend wurde viermal hintereinander 14,5 g (0,09 Mol) Carbonsäurechlorid innerhalb 15 min zugetropft, 5 min nachgerührt und mit 20 %iger Natronlauge auf pH 5,5 gestellt.

Nach der letzten Natronlaugezugabe wurde 1 Stunde nachgerührt, mit konz. HCl auf pH = 2,5 eingestellt, auf 80°C erhitzt, mit konz. HCl auf pH = 1,5 eingestellt und 1 Stunde nachgerührt.

Es wurde innerhalb von 2 Stunden auf 0°C gekühlt, abgesaugt und bei Raumtemperatur mit 2 x 100 ml Wasser gewaschen.

Nach dem Trocknen erhielt man in 70 %iger Ausbeute (bezogen auf eingesetztes 2,3-Dichlornitrobenzol) 1-Methylcyclohexancarbonsäure-(4-hydroxy-2,3-dichlor)anilid.

### Beispiel 2

Die Reaktionsfolge aus Beispiel 1 wurde wiederholt, wobei statt 1-Methylcyclohexancarbonsäurechlorid 22,9 g und 4 x 10,9 g Pivaloylchlorid eingesetzt wurde. Man erhielt in 72 %iger Ausbeute (bezogen auf eingesetztes 2,3-Dichlornitrobenzol) Pivaloylsäure-(4-hydroxy-2,3-dichlor)anilid.

### Beispiel 3

In einem 1,3 l Emailleautoklaven werden 42,9 g (0,276 Mol) 2-Chlor-3-nitrotoluol, 80 ml Dimethoxyethan, 400 ml H₂O, 30 g H₂SO₄ (98 %ig = 0,3 Mol) sowie 2 g 5 % Pt/C vorgelegt und unter starkem Rühren auf 100°C erhitzt.

Dann wurde 10 bar Wasserstoff aufgedrückt und bis zur Druckkonstanz hydriert.

Die Reaktionsmischung wurde heiß filtriert, am Rotationsverdampfer auf 300 ml eingeengt und in einem Vierhalskolben mit Rührer, Rückflußkühler, Tropftrichter und pH-Meßelektrode übergeführt.

Es wurde mit 20 %iger Natronlauge auf pH 5,5 gestellt, mit 100 ml Toluol versetzt und auf 50°C erwärmt.

Es wurden 15,8 g (0,1 Mol) 1-Methylcyclohexancarbonsäurechlorid innerhalb von 20 min zugetropft, 5 min nachgerührt und mit 20 %iger Natronlauge wieder pH 5,5 eingestellt.

Anschließend wurde in vier Schritten jeweils 7,2 g (0,045 Mol) Carbonsäurechlorid innerhalb von 10 min zugetropft, 3 min nachgerührt und mit 20 %iger Natronlauge auf pH 5,5 gestellt.

Nach der letzten Natronlaugezugabe wurde 30 min nachgerührt, mit konz. HCl pH 2,5 eingestellt, auf 80°C erhitzt, mit konz. HCl pH 1,5 eingestellt und 30 min nachgerührt.

Innerhalb von 2 Stunden wurde auf 0°C gekühlt, abgesaugt und bei Raumtemperatur mit 3 x 50 ml Wasser gewaschen.

Nach dem Trocknen erhielt man 77 % 1-Methylcyclohexancarbonsäure-(2-chlor-3-methyl-4-hydroxy)anilid.

## Patentansprüche

1. Verfahren zur Herstellung von N-acylierten p-Aminophenolen der allgemeinen Formel 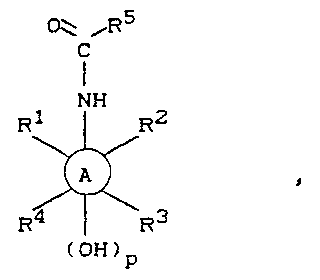 in der
A für den Benzol- oder den Naphthalinkern steht,
p die p-Stellung zur Aminogruppe anzeigt,
R¹, R², R³ und R⁴ unabhängig voneinander Wasserstoff, C₁-C₄-Alkyl, C₁-C₄-Alkoxy, Halogen oder die Gruppe CO-R⁶ bedeuten, in welcher R⁶ für Wasserstoff, C₁-C₄-Alkyl, C₁-C₄-Alkoxy, Hydroxy oder gegebenenfalls substituiertes Aryl steht, und
R⁵ C₁-C₂₀-Alkyl, C₃-C₁₀-Cycloalkyl, C₅-C₁₀-Cycloalkenyl, C₁-C₁₀-Alkoxy, gegebenenfalls substituiertes Aryloxy oder gegebenenfalls substituiertes Aryl bedeutet,
dadurch gekennzeichnet, daß Nitroverbindungen der allgemeinen Formel 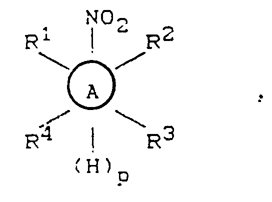 in der
R¹ bis R⁴, A und p die obige Bedeutung besitzen,
in einem wäßrig-sauren Reaktionsmedium, in welchem die Wassermenge das 2- bis 40-fache, bevorzugt das 3- bis 30-fache, besonders bevorzugt das 4- bis 20-fache der Gewichtsmenge der aromatischen Nitroverbindung beträgt, und gegebenenfalls in Gegenwart eines mit Wasser mischbaren organischen Lösungsmittels unter Zuhilfenahme eines Katalysators aus der Gruppe der Platinmetalle im Sinne einer Bamberger-Reaktion bei einer Temperatur von 50-160°C, bevorzugt 70-140°C, besonders bevorzugt 90-120°C, und einem Wasserstoff-Partialdruck von 0,1-50 bar, bevorzugt 0,5-30 bar, besonders bevorzugt 1-20 bar, hydriert werden und das erhaltene Reaktionsgemisch mit einer Verbindung der Formel 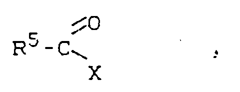 wobei
R⁵ die obige Bedeutung hat und
X für eine Fluchtgruppe steht,
in einer Menge von 0,5-2 Mol, bevorzugt 0,8-1,4 Mol pro Mol der eingesetzten Nitroverbindung, bei einer Temperatur von -30°C bis +150°C, bevorzugt von -20°C bis +120°C, besonders bevorzugt von -10°C bis +100°C, und gegebenenfalls in Gegenwart eines basischen Hilfsmittels oder eines anderen Säureakzeptors und gegebenenfalls in Gegenwart eines Verdünnungsmittels umsetzt.

2. Verfahren nach Anspruch 1, dadurch gekennzeichnet, daß die Reste R¹, R², R³, R⁴ und R⁶ unabhängig voneinander Wasserstoff, Methyl, Ethyl, Methoxy oder Ethoxy bedeuten, wobei die Reste R¹ bis R⁴ zusätzlich Fluor, Chlor oder Brom oder die Gruppe CO-R⁶ bedeuten kann und der Rest R⁶ zusätzlich Hydroxy oder gegebenenfalls substituiertes Phenyl bedeuten kann.

3. Verfahren nach Anspruch 1, dadurch gekennzeichnet, daß der Rest R⁵ C₁-C₈-Alkyl, C₃-C₆-Cycloalkyl, C₅-C₈-Cycloalkenyl, C₂-C₈-Alkoxy, gegebenenfalls substituiertes Phenoxy oder gegebenenfalls substituiertes Phenyl bedeutet.

4. Verfahren nach Anspruch 1, dadurch gekennzeichnet, daß die Fluchtgruppe X die Bedeutung Fluor, Chlor Brom, O-R⁵ oder O-CO-R⁵, bevorzugt die Bedeutung Chlor, O-R⁵ oder O-CO-R⁵ hat, wobei R⁵ den in Anspruch 1 gegebenen Bedeutungsumfang hat.

5. Verfahren nach Anspruch 1, dadurch gekennzeichnet, daß als organisches, mit Wasser mischbares Lösungsmittel eines oder mehrere aus der Gruppe der niederen Alkohole und Polyole mit 1 bis 4 C-Atomen, der Mono- und Dimethyl- und -ethylether solcher Polyole, der wasserlöslichen cyclischen Ether, der wasserlöslichen Ketone, der wasserlöslichen niederen Carbonsäureamide und der niederen aliphatischen Carbonsäuren mit 1 bis 4 C-Atomen, bevorzugt aus der Gruppe der niederen Alkohole, des Ethylenglykols und seiner Mono- und Dimethyl- und -ethylether und des Dioxans, besonders bevorzugt aus der Gruppe Methanol, Ethanol, Ethylenglykol, Glykol-mono- und -dimethylether und Dioxan, eingesetzt wird (werden), wobei die Menge des organischen, mit Wasser mischbaren Lösungsmittels das 0,01- bis 3-fache, bevorzugt das 0,03- bis 2-fache, besonders bevorzugt das 0,05- bis 1-fache der Gewichtsmenge der aromatischen Nitroverbindung beträgt.

6. Verfahren nach Anspruch 1, dadurch gekennzeichnet, daß Schwefelsäure oder eine organische Sulfonsäure, bevorzugt Schwefelsäure, in einer Menge von 0,4 bis 10 Äquivalenten, bevorzugt 0,5 bis 2 Äquivalenten, besonders bevorzugt 0,5 bis 1 Äquivalent pro Mol der aromatischen Nitroverbindung eingesetzt wird.

7. Verfahren nach Anspruch 1, dadurch gekennzeichnet, daß bei der Durchführung des 2. Reaktionsschrittes des erfindungsgemäßen Verfahrens als Verdünnungsmittel inerte organische Lösungsmittel wie aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid eingesetzt werden.

8. Verfahren nach Anspruch 1, dadurch gekennzeichnet, daß bei der Durchführung des 2. Reaktionsschrittes des erfindungsgemäßen Verfahrens ein basisches Hilfsmittel eingesetzt wird, bevorzugt Alkalimetall- oder Erdalkalimetallhydroxide, - carbonate oder -hydrogencarbonate oder tertiäre Amine,

9. Verfahren nach Anspruch 1, dadurch gekennzeichnet, daß als Nitroverbindung eine aus der Gruppe von Nitrobenzol, 2-Nitrotoluol, 3-Nitrotoluol, 2-Chlor-nitrobenzol, 3-Chlor-nitrobenzol, 2-Ethyl-nitrobenzol, 3-Ethyl-nitrobenzol, 2-Acetyl-nitrobenzol, 3-Acetyl-nitrobenzol, 2-Nitrobenzoesäure und Ester, 3-Nitrobenzoesäure und Ester, 2-Fluor-nitrobenzol, 3-Fluor-nitrobenzol, 2,3-Dichlor-nitrobenzol, 2,3-Difluor-nitrobenzol, 2-Chlor-3-fluor-nitrobenzol, 2-Fluor-3-Chlor-nitrobenzol, 1-Nitronaphthalin, 2-Chlor-3-methyl-nitrobenzol, 2-Methyl-3-chlor-nitrobenzol eingesetzt wird.

## Claims

1. Process for the preparation of N-acylated p-aminophenols of the general formula 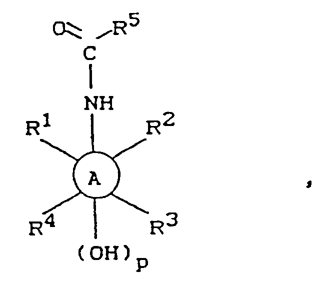 in which
A represents a benzene or naphthalene nucleus,
P indicates the p position to the amino group,
R¹, R², R³ and R⁴, independently of each other, denote hydrogen, C₁-C₄-alkyl, C₁-C₄-alkoxy, halogen or the group CO-R⁶ in which R⁶ represents hydrogen, C₁-C₄-alkyl, C₁-C₄-alkoxy, hydroxyl or unsubstituted or substituted aryl, and
R⁵ denotes C₁-C₂₀-alkyl, C₃-C₁₀-cycloalkyl, C₅-C₁₀-cycloalkenyl, C₁-C₁₀-alkoxy, unsubstituted or substituted aryloxy or unsubstituted or substituted aryl,
characterised in that nitro compounds of the general formula 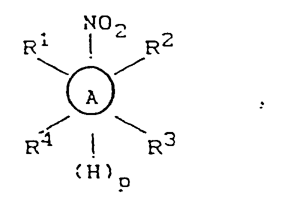 in which
R¹ to R⁴, A and p have the above meanings,
are hydrogenated in an aqueous acidic reaction medium in which the amount of water is 2 to 40 times, preferably 3 to 30 times, particularly preferably 4 to 20 times, the amount by weight of the aromatic nitro compound, and in the absence or presence of a water-miscible organic solvent with the aid of a catalyst selected from the group comprising the platinum metals in accordance with a Bamberger reaction at a temperature of 50-160°C, preferably 70-140°C, particularly preferably 90-120°C and a hydrogen partial pressure of 0.1-50 bar, preferably 0.5-30 bar, particularly preferably 1-20 bar and the resulting reaction mixture is reacted with a compound of the formula 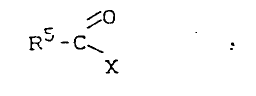 where
R⁵ has the above meaning and
X represents a leaving group,
in an amount of 0.5-2 mol, preferably 0.8-1.4 mol, per mol of the nitro compound used, at a temperature of -30°C to +150°C, preferably -20°C to +120°C, particularly preferably -10°C to +100°C, and in the absence or presence of a basic auxiliary or another acid acceptor and in the absence or presence of a diluent.

2. Process according to Claim 1, characterised in that the radicals R¹, R², R³, R⁴ and R⁶, independently of each other, denote hydrogen, methyl, ethyl, methoxy or ethoxy, where the radicals R¹ to R⁴ can additionally denote fluorine, chlorine or bromine or the group CO-R⁶ and the radical R⁶ can additionally denote hydroxyl or unsubstituted or substituted phenyl.

3. Process according to Claim 1, characterised in that the radical R⁵ denotes C₁-C₈-alkyl, C₃-C₆-cycloalkyl, C₅-C₈-cycloalkenyl, C₂-C₈-alkoxy, unsubstituted or substituted phenoxy or unsubstituted or substituted phenyl.

4. Process according to Claim 1, characterised in that the leaving group X has the meaning fluorine, chlorine, bromine, O-R⁵ or O-CO-R⁵, preferably the meaning chlorine, O-R⁵ or O-CO-R⁵, where R⁵ has the range of meanings given in Claim 1.

5. Process according to Claim 1, characterised in that the organic, water-miscible solvent(s) used is(are) one or more selected from the group comprising the lower alcohols and polyhydric alcohols having 1 to 4 C atoms, the monomethyl, dimethyl, monoethyl and diethyl ethers of such polyhydric alcohols, the water-soluble cyclic ethers, the water-soluble ketones, the water-soluble lower carboxylic amides and the lower aliphatic carboxylic acids having 1 to 4 C atoms, preferably selected from the group comprising the lower alcohols, ethylene glycol and its monomethyl, dimethyl, monoethyl and diethyl ethers and dioxane, particularly preferably selected from the group comprising methanol, ethanol, ethylene glycol, glycol monomethyl ether and glycol dimethyl ether and dioxane, where the amount of the organic, water-miscible solvent is 0.01 to 3 times, preferably 0.03 to 2 times, particularly preferably 0.05 to 1 times the amount by weight of the aromatic nitro compound.

6. Process according to Claim 1, characterised in that sulphuric acid or an organic sulphonic acid, preferably sulphuric acid, is used in an amount of 0.4 to 10 equivalents, preferably 0.5 to 2 equivalents, particularly preferably 0.5 to 1 equivalent, per mol of the aromatic nitro compound.

7. Process according to Claim 1, characterised in that when the 2nd reaction step of the process according to the invention is carried out, the diluents used are inert organic solvents such as aliphatic, alicyclic or aromatic, unhalogenated or halogenated hydrocarbons such as benzine, benzene, toluene, xylene, chlorobenzene, petroleum ether, hexane, cyclohexane, dichloromethane, chloroform, carbon tetrachloride, ethers such as diethyl ether, dioxane, tetrahydrofuran or ethylene glycol dimethyl ether or ethylene glycol diethyl ether, ketones such as acetone or butanone, nitriles such as acetonitrile or propionitrile, amides such as dimethylformamide, dimethylacetamide, N-methylformanilide, N-methylpyrrolidone or hexamethylphosphoric triamide, esters such as ethyl acetate or sulphoxides such as dimethyl sulphoxide.

8. Process according to Claim 1, characterised in that, when the 2nd reaction step of the process according to the invention is carried out, a basic auxiliary is used, preferably alkali metal hydroxides or alkaline earth metal hydroxides, alkali metal carbonates or hydrogen carbonates or alkaline earth metal carbonates or hydrogen carbonates, or tertiary amines.

9. Process according to Claim 1, characterised in that the nitro compound used is one selected from the group comprising nitrobenzene, 2-nitrotoluene, 3-nitrotoluene, 2-chloronitrobenzene, 3-chloronitrobenzene, 2-ethyl-nitrobenzene, 3-ethylnitrobenzene, 2-acetyl-nitrobenzene, 3-acetylnitrobenzene, 2-nitrobenzoic acid and esters, 3-nitrobenzoic acid and esters, 2-fluoro-nitrobenzene, 3-fluoro-nitrobenzene, 2,3-dichloro-nitrobenzene, 2,3-difluoro-nitrobenzene, 2-chloro-3-fluoronitrobenzene, 2-fluoro-3-chloro-nitrobenzene, 1-nitronaphthalene, 2-chloro-3-methyl-nitrobenzene, 2-methyl-3-chloro-nitrobenzene.

## Revendications

1. Procédé pour préparer des p-aminophénols N-acylés de formule générale : 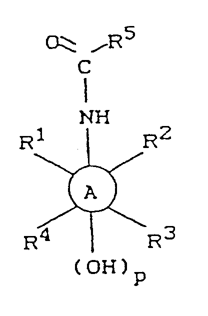 dans laquelle
A représente le noyau benzénique ou naphtalénique,
p indique la position para par rapport au groupe amino
R¹, R², R³ et R⁴ représentent chacun, indépendamment l'un de l'autre, un atome d'hydrogène, un groupe alkyle en C₁-C₄, alcoxy en C₁-C₄, un atome d'halogène ou le groupe CO-R⁶, dans lequel R⁶ représente un atome d'hydrogène, un groupe alkyle en C₁-C₄, alcoxy en C₁-C₄, hydroxy ou un groupe aryle éventuellement substitué, et
R⁵ représente un groupe alkyle en C₁-C₂₀, cycloalkyle en C₃-C₁₀, cycloalcényle en C₅-C₁₀, alcoxy en C₁-C₁₀, un groupe aryloxy éventuellement substitué ou un groupe aryle éventuellement substitué,
procédé caractérisé en ce qu'on soumet à hydrogénation des composés nitrés de formule générale : 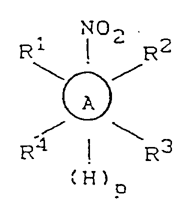 dans laquelle
R¹ à R⁴, A et p ont le sens précité, dans un milieu réactionnel acide aqueux, dans lequel la quantité d'eau représente de 2 à 40 fois, de préférence de 3 à 30 fois, de façon particulièrement préférée de 4 à 20 fois la quantité pondérale du composé nitré aromatique, et en opérant éventuellement en présence d'un solvant organique miscible à l'eau, à l'aide d'un catalyseur choisi dans l'ensemble formé par les métaux de la famille du platine, au sens d'une réaction de Bamberger, à une température de 50 à 160°C, de préférence 70 à 140°C, de façon particulièrement préférée 90 à 120°C, et sous une pression partielle d'hydrogène de 0,1 à 50 bars, de préférence de 0,5 à 30 bars, de façon particulièrement préférée 1 à 20 bars, et l'on fait réagir le mélange réactionnel ainsi obtenu avec un composé de formule : 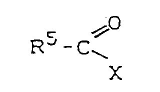 dans laquelle
R⁵ a le sens précité, et
X représente un groupe partant ou volatil, en une quantité de 0,5 à 2 mol, de préférence 0,8 à 1,4 mol par mole du composé nitré utilisé, à une température de -30°C à +150°C, de préférence de -20°C à +120°C, de façon particulièrement préférée entre -10°C et +100°C, et éventuellement en présence d'un adjuvant basique ou d'un autre accepteur d'acide et éventuellement en présence d'un diluant.

2. Procédé selon la revendication 1, caractérisé en ce que les restes R¹, R², R³, R⁴ et R⁶ représentent chacun, indépendamment l'un de l'autre, un atome d'hydrogène, un groupe méthyle, éthyle, méthoxy ou éthoxy, des restes R¹ à R⁴ pouvant représenter en outre le fluor, le chlore ou le brome ou le groupe CO-R⁶ et le reste R⁶ pouvant représenter également un groupe hydroxy ou un groupe phényle éventuellement substitué.

3. Procédé selon la revendication 1, caractérisé en ce que le reste R⁵ représente un groupe alkyle en C₁-C₈, cycloalkyle en C₃-C₆, cycloalcényle en C₅-C₈, alcoxy en C₂-C₈, phénoxy éventuellement substitué ou phényle éventuellement substitué.

4. Procédé selon la revendication 1, caractérisé en ce que le groupe volatil X représente le fluor, le chlore ou le brome, O-R⁵ ou O-CO-R⁵, de préférence le chlore, ou O-CO-R⁵, le symbole R⁵ ayant le sens indiqué à la revendication 1.

5. Procédé selon la revendication 1, caractérisé en ce qu'on utilise comme solvant organique miscible à l'eau un ou plusieurs solvants choisis dans l'ensemble formé par les alcools et polyols inférieurs comportant 1 à 4 atomes de carbone, l'éther monométhylique et l'éther diméthylique et l'éther monoéthylique et diéthylique de tels polyols, les éthers cycliques hydrosolubles, les cétones hydrosolubles, les carboxamides inférieurs hydrosolubles et les acides carboxyliques aliphatiques inférieurs comportant 1 à 4 atomes de carbone, de préférence un solvant choisi dans l'ensemble formé par des alcools inférieurs, l'éthylèneglycol et ses éthers monométhylique, diméthylique et monoéthylique et diméthylique et le dioxane, de façon particulièrement préférée un diluant choisi dans l'ensemble formé par le méthanol, l'éthanol, l'éthylèneglycol, l'éther monobutylique et l'éther diméthylique du glycol et le dioxane, la quantité du solvant organique miscible à l'eau représentant 0,01 à 3 fois, de préférence 0,03 à 2 fois, de façon particulièrement préférée 0,05 à 1 fois la quantité pondérale du composé nitré aromatique.

6. Procédé selon la revendication 1, caractérisé en ce qu'on utilise l'acide sulfurique ou un acide sulfonique organique, de préférence l'acide sulfurique, en une quantité de 0,4 à 10 équivalents, de préférence 0,5 à 2 équivalents, de façon particulièrement préférée, 0,5 à 1 équivalent par mole du composé nitré aromatique.

7. Procédé selon la revendication 1, caractérisé en ce que, lors de la réalisation de la seconde étape de réaction du procédé selon l'invention, on utilise comme diluant des solvants organiques inertes comme des hydrocarbures aliphatiques, alicycliques ou aromatiques, éventuellement halogénés, comme par exemple l'essence, le benzène, le toluène, le xylène, le chlorobenzène, l'éther de pétrole, l'hexane, le cyclohexane, le dichlorométhane, le chloroforme, le tétrachlorure de carbone, des éthers comme l'éther diéthylique, le dioxane, le tétrahydrofurane ou l'éther diméthylique ou diéthylique de l'éthylèneglycol, des cétones, comme l'acétone ou la butanone, des nitriles comme l'acétonitrile ou le propionitrile, des amides comme le diméthylformamide, le diméthylacétamide, le N-méthylformanilide, la N-méthylpyrrolidone ou le triamide hexaméthylique de l'acide phosphorique, des esters comme l'acétate d'éthyle ou des sulfoxydes comme le diméthylsulfoxyde.

8. Procédé selon la revendication 1, caractérisé en ce que, lors de la conduite de la seconde étape de réaction du procédé selon l'invention, on utilise un adjuvant basique, de préférence des hydroxydes, carbonates ou hydrogénocarbonates de métaux alcalins ou de métaux alcalino-terreux, ou des amines tertiaire.

9. Procédé selon la revendication 1, caractérisé en ce qu'on utilise comme composé nitré un composé choisi dans l'ensemble formé par des nitrobenzènes, le 2-nitrotoluène, le 3-nitrotoluène, le 2-chloronitrobenzène, le 3-chloronitrobenzène, le 2-éthylnitrobenzène, le 3-éthylnitrobenzène, le 2-acétylnitrobenzène, le 3-acétylnitrobenzène, l'acide 2-nitrobenzoïque et ses esters, l'acide 3-nitrobenzoïque et ses esters, le 2-fluoronitrobenzène, le 3-fluoronitrobenzène, le 2,3-dichloronitrobenzène, le 2,3-dichloronitrobenzène, le 2-chloro-3-fluoronitrobenzène, le 2-fluoro-3-chloro-nitrobenzène, le 1-nitronaphtalène, le 2-chloro-3-méthylnitrobenzène, le 2-méthyl-3-chloronitrobenzène.
